# EUROPEAN PATENT SPECIFICATION

(11) **EP 1 691 199 B1**
(45) Date of publication and mention of the grant of the patent: **01.04.2009**
(21) Application number: 04773660.8
(22) Date of filing: 30.09.2004
(51) Int. Cl.: C12Q 1/68, G01N 33/68, G01N 33/50, G01N 33/15

(54) **METHOD OF PREDICTING SPOT FORMATION ON THE SKIN WITH THE USE OF SPOT SITE-ACCELERATING GENES AS INDICATION AND METHOD OF SCREENING INHIBITOR FOR SPOT FORMATION ON THE SKIN**
VERFAHREN ZUR VORHERSAGE DER PICKELBILDUNG AUF DER HAUT UNTER VERWENDUNG VON PICKELSTELLEN BESCHLEUNIGENDEN GENEN ZUR ANZEIGE SOWIE SCREENING-VERFAHREN FÜR EINEN INHIBITOR GEGEN PICKELBILDUNG AUF DER HAUT
PROCEDE DE PREVISION DE LA FORMATION DE TACHES SUR LA PEAU A L'AIDE DE GENES D'ACCELERATION DE SITES DE TACHES SERVANT D'INDICATEURS ET PROCEDE DE CRIBLAGE D'INHIBITEURS DE LA FORMATION DE TACHES SUR LA PEAU

(30) Priority: 01.10.2003 JP 2003343549; 02.10.2003 JP 2003344786
(43) Date of publication of application: 16.08.2006
(73) Proprietor: SHISEIDO COMPANY LIMITED, Chuo-ku, Tokyo 104-8010 (JP)
(72) Inventor: AOKI, Hirofumi, c/o SHISEIDO RESEARCH CENTER, Yokohama-shi, Kanagawa 224-8558 (JP); KODAMA, Tatsuhiko, Setagaya-ku Tokyo 1540002 (JP); HASEGAWA, Kiyotaka, c/o SHISEIDO RESEARCH CENTER, Yokohama-shi, Kanagawa 224-8558 (JP); KAJIYA, Kentaro, c/o SHISEIDO RESEARCH CENTER, Yokohama-shi, Kanagawa 236-8643 (JP); ISHIMATSU, Yumiko, c/o SHISEIDO RESEARCH CENTER, Yokohama-shi, Kanagawa 224-8558 (JP); OGOU, Masashi, c/o SHISEIDO COMPANY LTD., Chuo-ku, Tokyo 104-8010 (JP); YOSHIDA, Seiichi, c/o SHISEIDO RESEARCH CENTER, Yokohama-shi, Kanagawa 224-8558 (JP); KISHIMOTO, Jiro, c/o SHISEIDO RESEARCH CENTER, Yokohama-shi, Kanagawa 224-8558 (JP); MORO, Osamu, c/o SHISEIDO RESEARCH CENTER, Yokohama-shi, Kanagawa 224-8558 (JP)
(74) Representative: Ulmann, Catherine Claire
(86) International application number: PCT/JP2004/014799
(87) International publication number: WO 2005/033710

(56) References cited:
- WO-A-02/22888
- WO-A2-02/43758
- JP-A- 2003 245 097
- JP-A- 2003 271 728
- JP-A- 2004 205 246
- US-A1- 2002 111 290
- KADONO SATSUKI ET AL: "The role of the epidermal endothelin cascade in the hyperpigmentation mechanism of lentigo senilis" JOURNAL OF INVESTIGATIVE DERMATOLOGY, vol. 116, no. 4, April 2001 (2001-04), pages 571-577, XP002460364 ISSN: 0022-202X
- AOKI H ET AL: "Analysis of gene expression of solar lentigines like spots in model" PIGMENT CELL RESEARCH, MUNKSGAARD, COPENHAGEN, DK, vol. 15, no. suppl 9, 2002, page 72, XP009092724 ISSN: 0893-5785
- BOSSET S ET AL: "Decreased expression of keratinocyte beta1 integrins in chronically sun-exposed skin in vivo." BRITISH JOURNAL OF DERMATOLOGY, vol. 148, no. 4, April 2003 (2003-04), pages 770-778, XP002460365 ISSN: 0007-0963
- DISTLER O ET AL: "Monocyte chemoattractant protein (MCP) -1, -2 and -3 in the skin of patients with progressive systemic sclerosis (SSc)" ARTHRITIS AND RHEUMATISM, vol. 41, no. 9 SUPPL., September 1998 (1998-09), page S322, XP009092948 & 62ND NATIONAL SCIENTIFIC MEETING OF THE AMERICAN COLLEGE OF RHEUMATOLOGY AND THE 33RD NATIONAL SCIEN; SAN DIEGO, CALIFORNIA, USA; NOVEMBER 8-12, 1998 ISSN: 0004-3591

### SEQUENCE LISTING

<110> Shiseido Co. Ltd.
   <120> Methods for Predicting Skin Stain Formation and for Screening Agent Capable of Inhibiting Skin Stain Formation By Means of Promoted Genes in S tained Regions as an Indicator
   <130> P871
<150> JP 2003-343549
   <151> 2003-10-01
<150> JP 2003-344786
   <151> 2003-10-02
<160> 7
   <210> 1
   <211> 758
   <212> DNA
   <213> Mouse
   <400> 1
   aaaccntttt cgtggcacag cttcctccct aggcgtgaga ctccggctcc ttcactatga 60
   gacttctagc cctttccggt ctgctctgca tgctgctcct ctgtttctgc attttctcct 120
   cagaagggag aagacatcct gccaagtcct tgaaactcag gcgctgctgt cacctatctc 180
   ctagatccaa gctgacaacc tggaaaggaa accacacaag gccctgcaga ctctgcagaa 240
   acaagctacc agtcaagtca tgggtggtgc ctggggctct cccacagata tagggcctcc 300
   tccgcccaga tgaagcgttg atgcccagat gtggagacac cagaagcata cacactatgt 360
   tgccttgccc cttgccaatg agctgtgaca ctggaatgct tcacttcaga catcagggcg 420
   gatggattgc agaattccaa gtcctcattc caaaggtgtc accaaccttc agagtcacta 480
   aggtccaggc tcagcccaca agtcaccatg gctcctccag agtaaaagtc caagattcca 540
   cctgtgggag ctacagatcc agagactttc aagctgacta gagtgcagag aagcaagacc 600
   tcagtgtgat cagccgagac tacagcatct tgggaaccct cagtcagccc caaaccccta 660
   acacttaacc actggtctcc aaaccaacac ctgtaacttc ctaatgaaat catcaggagg 720
   ataccaaaag aaataaacca taaatcagca tacacacg 758
<210> 2
   <211> 2063
   <212> DNA
   <213> Homo Sapiens
   <400> 2
   ctctgcccat tgggaaacac ctctctatga ctctataaat gtccaaggtg gccccaaggg 60
   aggacttctg cagcacagct cccttcccag gacgtgaaaa tctgccttct caccatgagg 120
   cttctagtcc tttccagcct gctctgtatc ctgcttctct gcttctccat cttctccaca 180
   gaagggaaga ggcgtcctgc caaggcctgg tcaggcagga gaaccaggct ctgctgccac 240
   cgagtcccta gccccaactc aacaaacctg aaaggacatc atgtgaggct ctgtaaacca 300
   tgcaagcttg agccagagcc ccgcctttgg gtggtgcctg ggcactcccc acaggtgtag 360
   cactcccaaa gcaagactcc agacagcgga gaacctcatg cctggcacct gaggtaccca 420
   gcagcctcct gtctcccctt tcagccttca cagcagtgag ctgcaatgtt ggagggcttc 480
   atctcgggct gcaaggaccc tgggaaagtt ccagaactcc acgtccttgt ctcaattgtg 540
   ccatcaactt tcagagctat catgagccaa cctcagcctt ccgagtagct gggattacag 600
   gtgtgcgcca ccacacccgg ctaatttttg cttttttttt ttttgagaca gaatctcgct 660
   ctgttgctca ggctggagtg gagtgcagtg gcacgatctc agctcactgc agcctctgcc 720
   tcccgggttc aggagattct cctgactcaa cctcctgagt agctgggatt acaggtgcct 780
   gccactatgc ctagctaatt tttgtatttt tagtggagac ggggttttgc cgtgttggcc 840
   ggggtggtct cgaactcctg acctcaagtg atccacccgc atcggccccc caaagtgctg 900
   ggattatagg cgtgaaccac cgcgcctgtc ccattgttgt gtaattttaa taattagttt 960
   tttaagtact tgattttatg ggcacatttt tgtgggatga ttggagttaa tcaaataaag 1020
   cttgtcatgt gtgtagtttg gtaagataac ttctttaaat tcatgttttc tctgccttga 1080
   ggtagtgagg gaaagatctt aatcagtatt ttggtaatta actgattgaa ttcaagcaaa 1140
   tgagacatca tgaacttcag tggttattga tatttcaggg tatatacctg aaatgcctag 1200
   aggatacaga tttctcattt cattctttgg tctttcattt ctctatatac agaaatgaaa 1260
   tgacacttct gggaggcagt agaagcagga agtcaatgaa ttgagtagag ggtcccattc 1320
   cctcaggctg tcattgatca gtgacaattt ataaaaacaa actgcaaagt ctgtggcaag 1380
   tggctgcctg cttcctagaa ggagcccatg aaggttaaac tctgtggtcg gtatttgcaa 1440
   gcgccgggcg tggtggctca cgcctgtaat cctagcacct tgggaggcca aggcaggcgg 1500
   atcacccgag gtcaggagtt tgaggatttg caagcaaaag gtcctctcct gagtctttcc 1560
   cagataccca gcagtgcaga ggctagctgt ggaaggttgc agtgggacag gaatgtattg 1620
   tatgccttgc cttacttgtc accattgaga tttccagaga aatgggcata acgtctctta 1680
   acaacaacag cagaaagcaa aatacattaa cttaaggttg acaacaaaag attatcaagt 1740
   accatgtttt ccaaccaacc agttattcgt ggtaataata aaataaaggt gggaaaatgt 1800
   tataattttt aaggaaactg tgtactttaa aaatcttctt tatgaatatc caatgttact 1860
   gtaatcctgc tccattaaat gcagcattgt tgtcaggtgc tgcctcttgc ttgggaacag 1920
   cattgggctt ttaaatgtct gcagaatctc tgcgttcgaa gggaattgag aatgaacttc 1980
   ctggtactgt aatgaaaata aggtctgctc aacacagtaa acgtttcctc tcttctttaa 2040
   aaaaaaaaaa aaaaaaaaaa aaa 2063
<210> 3
   <211> 742
   <212> DNA
   <213> Rat
   <400> 3
   tttttttgtg ccactgcttc ctctctagcc gtgagactcc agctacttca ctatgcgact     60
   tctcaccctc tccggtttgt tcttcatgct gttcctctgt ctctgcgttc tctcctcaga    120
   agggagaaag cgtcctgcca agttcccgaa actcaggcct gctgtcatct atctcctaga    180
   tccaaaccaa taactggaaa ggaaaccaca caagaccctg cagaccatgc agaaagctag    240
   aatccaattc atggggtggt gcctggggct ctcccacaga tatagggcct cccgaagctg    300
   gcctccaccg agatgaaacg ttgatgtcca gttatggaga caaccttctg gcccctacca    360
   accttcatgg ccagaaagct gtgacaccag aatgtttcac ttcagacagc tgaaggatta    420
   cagaattcca agccctcgtt ccaaaggtgc aaccaacctt cagagtcact atgatccagg    480
   gtcagcccac aagtcttcat ggctcctgca gagtaaaagt ccaagattcc atccctggga    540
   gctacagatt cagagacttc caagctgact ggcgaacaga gtagcaagac ttccttgtga    600
   tcagatgaga ttacagcatc ttaggaaccc tcggacaccc ccaaacccat agcatttaat    660
   caacgggata tgaaccaact cctgtaactt cctaatgtaa tcaccaggag aacaccaaaa    720
   ataataaatc ataaatcaat gt                                             742
<210> 4
   <211> 20
   <212> DNA
   <213> Artificial Sequence
   <223> Sense Primer
<400> 4
   ttctttgcct gctgctcata 20
   <210> 5
   <211> 20
   <212> DNA
   <213> Artificial Sequence
   <223> Antisense Primer
   <400> 5
   gacaaggatg agaaaacacg 20
   <210> 6
   <211> 20
   <212> DNA
   <213> Artificial Sequence
   <223> Sense Primer
   <400> 6
   actccggctc cttcactatg 20
   <210> 7
   <211> 20
   <212> DNA
   <213> Artificial Sequence
   <223> Antisense Primer
   <400> 7
   ctttggaatg aggacttgga 20

## Description

### TECHNICAL FIELD

The present invention relates to a skin test method for predicting the formation of spots.

### BACKGROUND ART

When the action of the enzyme tyrosinase within melanocytes (pigment-forming cells) is activated abnormally due to ultraviolet rays, hormonal imbalance or psychological stress and so forth, formation of melanin pigment is enhanced and they are successively sent out to surrounding epidermal cells. If the rate at which melanin pigment is produced is excessively fast and turnover is no longer normal due to the effects of ultraviolet rays and so forth, the melanin pigment is unable to be excreted to the outside and remains in the skin, and this is believed to result in the formation of spots in the skin.

Once a spot has been formed, it is preferably to treat the spot as quickly as possible, and a visual sensory evaluation of the spot by a beautician, or an early assessment of the presence of a spot by a quantitative evaluation of the spot using equipment such as an apparatus for capturing images of skin condition or a colorimeter, is desired for the purpose of providing treatment (Japanese Unexamined Patent Publication No. 2003-144393).

Once a spot being formed, it is not easily removed, and treatment is required that improves skin metabolism to quickly expel the unnecessary melanin and prevent excess melanin from being formed. Thus, it is preferably to care for the skin prior to the formation of a spot. However, since there are individual differences in susceptibility to spot formation and there are various conditions that cause their formation, it is typically difficult to predict the formation of a skin spot. Accordingly, a means for predicting whether or not the skin is susceptible to spot formation prior to formation thereof would be extremely effective as a preventive measure.

### DISCLOSURE OF THE INVENTION

In consideration of the aforementioned problems, the inventors of the present invention conducted extensive studies on whether it is possible to provide a means for predicting whether or not the skin is susceptible to spot formation before a spot is formed in the skin. As a result of conducting a microarray analysis of epidermal RNA from spot sites and non-spot sites in spot model mice in which the skin was irradiated with ultraviolet rays after which irradiation was discontinued so that sunburn-like coloring was discolored followed by waiting briefly before forming pigment spots resembling age spots (M. Naganuma et al., Journal of Dermatological Science 25 (2001) 29-35), the inventors of the present invention found that, in comparison with non-spots sites that were not irradiated with ultraviolet rays and where spot-like pigment spots did not form, expression of the following genes was specifically increased in the epidermis of the spot sites. Thus, it was clearly demonstrated that whether or not skin is susceptible to the formation of spots can be assessed by investigating expression of the MCP-2 gene in human epidermis.

### (1) MCP-2 (Monocyte Chemoattracting Protein 2)

This protein is a type of chemokine (family of small cytokines having molecular weights of about 10,000). Although another member of this family, MCP-1, has been reported to cause non-tumor-forming melanoma cells to form tumors via attraction of monocytes (Journal Immunol. Jun 1; 166(11): 6483-6490), there are no reports on its correlation with skin pigment.

Further, it was clearly demonstrated that whether or not skin is susceptible to the formation of spots can be assessed by investigating expression of the following genes in human epidermis.

Any disclosure concerning these genes is not part of the present invention and is merely provided for information purposes.

### (2) AK012157 Gene (SEQ. ID NO. 1)

Only the base sequence of this gene is known, and there are no reports describing its function (Meth. Enzymol. 303, 19-44 (1999)). Although a gene having about 80% homology with this gene (S74257: SEQ. ID NO. 3) has been reported to be related to cancer invasion and metastasis in rats (Oncogene, 1994, 9 (12), 3591-3600), there are no reports on the correlation between this gene and skin pigment. Moreover, although a gene having about 70% homology with mouse gene AK012157 is known to exist in humans (human FLJ21763 gene (SEQ. ID.NO. 2)), there are also no reports describing the correlation between this gene and skin pigment.

### (3) Gene Group for which Expression is Known to be Increased by Interferon (hereinafter referred to as "Gene Group 1")

### a) Chemokine-Related Genes

Mcp9 (small inducible cytokine B subfamily (Cys-X-Cys), member 9) (Arthritis Rheum 2002 Oct; 46(10): 2730-41);
Mcp10 (small inducible cytokine B subfamily (Cys-x-Cys), member 10 (J Immunol. 2002 Apr 1; 168(7): 3195-204);

### b) Signal Transfer-Related Gene

Isg15 (Interferon-stimulated protein (15 kDa) isg15 (Ubiquitin-like)) (Genes Dev 2003 Feb 15; 17(4): 455-460);
Usp18 (ubiquitin specific protease 18) (J. Biol. Chem. 2002 Mar 22; 277(12): 9976-9981);

### c) Antivirus-Related Gene

Oas12 (2'-5'-oligoadenylate synthase-like OASL2 (IFN induced)) (J. Interferon Cytokine Res 2002 Sep; 22(9): 981-993);
Gbp2 (IFN induced guanylate nucleotide binding protein 2 gbp2 (antivirus)) (J. Interferon Cytokine Res 1998 Nov; 18(11): 977-985);
Gtpi (GTPase; interferon-g induced GTPase (19440);
Ifi97 (interferon gamma inducible protein, 47 kDa (GTP-binding motif) (J. Immunol. 1992 May 15; 148(10): 3275-81);
Igtp (GTPase; interferon gamma induced GTPase igtp) (Infect Immun. 2002 Dec; 70(12): 6933-9);
Tgtp (GTPase; T-cell specific GTPase (IFN gamma)) (J Leukoc Biol. 1995 Mar; 57(3): 477-83).

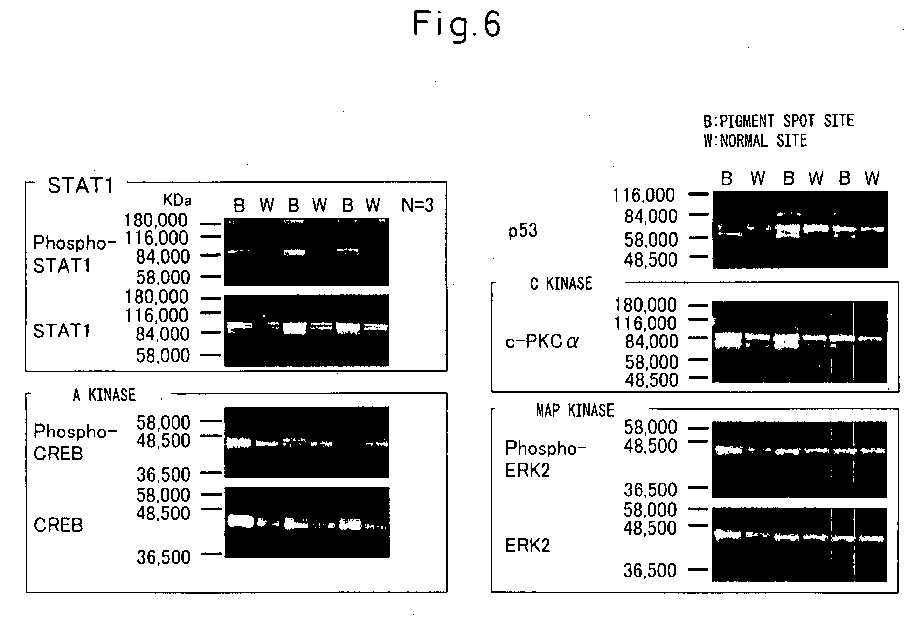

An interferon reactive element is present in the promoter region of genes for which expression is increased by interferon, and the expression of these genes is increased as a result of binding thereto STAT-1 (signal transducers and activators of transcription) being activated by phosphorylation (Free Radical Biology & Medicine 2000; 28(9): 1430-1437; Exp Dermatol 1999; 8: 96-108). Thus, the aforementioned Gene Group (1) can be said to be a gene group for which expression is increased in the presence of phosphorylated STAT-1 that has been activated by phosphorylation. As is actually shown in Fig. 6, phosphorylated STAT-1 has also been shown to be expressed at high levels at spot sites.

### (4) Other Genes Having Known-Functions (hereinafter to be referred to as "Gene Group 2")

### a) Keratin-Related Gene

Sprr2A (small proline-rich protein 2A) (Mamm. Genome 2003; 14(2): 140-148);
Krt2-6b (keratin complex 2, basic, gene 6a) (Genomics 1998; 53(2): 170-183);

### b) Cell Cycle-Related Gene

Cdk5rap2 (CKK5 regulatory subunit associated protein 2) (Neuron. 2003 Apr 10; 38(1): 33-46);
Mef2C (myocyte enhancer factor 2C) (Brain Res Mol Brain Res. 2001 Dec 16; 97(1): 70-82);

### c) Oxidation-Reduction-Related Gene

Gsta4 (glutathione S-transferase, alpha 4) (J. Biol. Chem. 2002 May 17; 227(20): 17892-17900);

### d) Bone-Related Gene

Osf2 (osteoblast specific factor (facilin I-like)) (Protein Expr Purif 1995 Jun; 6(39): 305-311);

### e) Extracellular matrix (ECM)-Related Gene

Tnc (Tenascin C) (Matrix Biol 2000 Dec; 19(7): 581-596);

### f) Insulin-Related Gene

Igfbp6 (Insulin-like growth factor binding protein 6) (Mol. Cell. Endocrinol. 1994; 104 (1) : 57-66) ;

### g) Cyclosporin-Related Gene

Ppicap (peptidylprolyl isomerase C (cyclophylin C)-associated protein) (Proc Natl Acad Sci USA. 1993 Jul 15; 90 (14): 6815-9);
MCP-6 (Mast cell protease 6) (J. Biol. Chem. 1991 Feb 25; 266(6): 3847-3853).

### (5) Unknown Function Gene Group (hereinafter to be referred to as "Gene Group 3")

Mm. 74656 Gene (GenBank Acc: Aa519023)

There are no reports describing a correlation with skin pigment for any of the gene groups of (3). Thus, it is extremely surprising that the expression of these genes is increased in association with spots.

In a first perspective of the invention, the present invention provides a skin test method for predicting the formation of spots. This method is characterized in that skin is judged to be susceptible to the formation of spots in the case expression of MCP2 in epidermis is increased as compared with normal expression in the epidermis.

In a preferable aspect thereof, the formation of spots is caused by UVB radiation.

In a more preferable aspect thereof, the increase in the expression of MCP2 in epidermis is determined by measuring the amount of MCP2 in the epidermis.

In a more preferable aspect thereof, the measurement is carried out by ELISA or RIA using antibody specific to MCP2 in the epidermis.

In a more preferable aspect thereof; the increase in the expression of MCP2 in the epidermis is determined by measuring the amount of mRNA encoding MCP2 extracted from the epidermis.

In a more preferable aspect thereof, the measurement of the mRNA is carried out by a polymerase chain reaction method.

In a second perspective thereof, the present invention provides a method for screening for a spot formation inhibitory factor and/or spot removal factor. This method is characterized by evaluating a candidate compound for the ability to inhibit the expression and/or activity in the epidermis of MCP2, and select an MCP2 inhibitor having this inhibitory ability as a spot formation inhibitory factor and/or spot removal factor.

In a preferable aspect thereof, this method further comprises the application of the MCP2 inhibitor having inhibitory ability to a spot formation model animal, and selecting an inhibitor that has a spot formation inhibitory effect and/or spot removal effect.

According to the present invention, a skin test method can be provided for predicting the formation of spots.

### BRIEF DESCRIPTION OF THE DRAWINGS

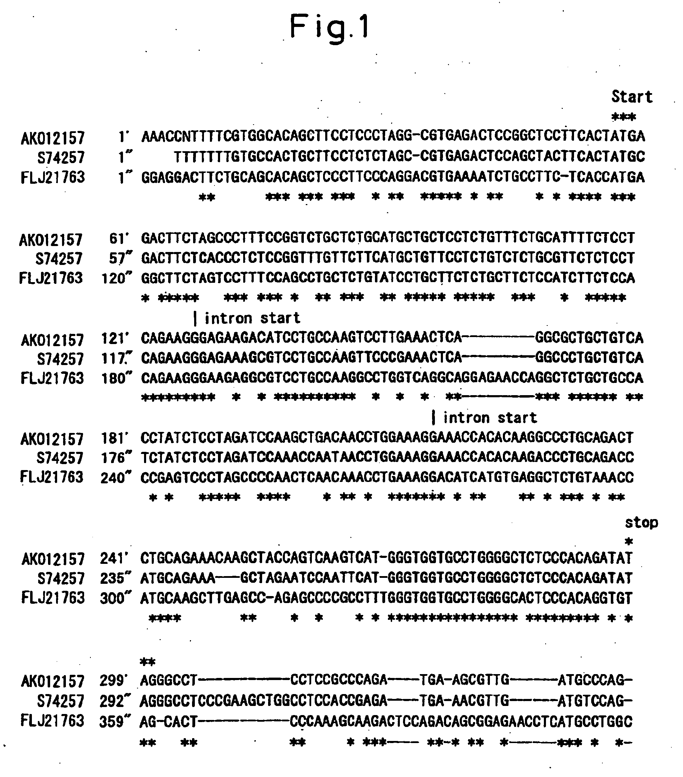

Fig. 1 is a comparison of mouse AK012157 gene, human FLJ21763 gene and rat S74257 gene.

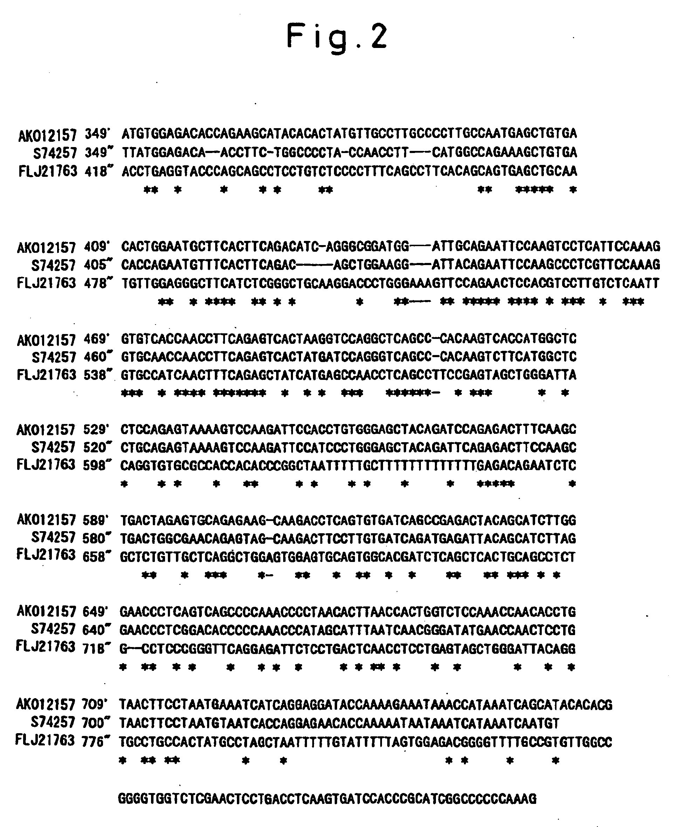

Fig. 2 is a continuation of Fig. 1.
Fig. 3 shows the expression of AK012157 gene and MCP-2 gene in the epidermis and dermis of spot model mice by PCR.
Fig. 4 shows the expression of AK012157 gene in the epidermis of spot model mice by in situ hybridization.
Fig. 5 shows the expression of MCP-2 in the epidermis of spot model mice by immunohistostaining.
Fig. 6 shows differences in the expression of major signal proteins between spot sites and normal sites as determined by Western blotting.

### BEST MODE FOR CARRYING OUT THE INVENTION

As has been described above, there have been no reports describing a correlation with skin pigment for mouse or human MCP-2. As a result of conducting microarray analyses on RNA originating in the epidermis of spot sites and normal sites of spot model mice subjected to ultraviolet radiation, the inventors of the present invention found that the expressions of MCP-2 gene was specifically increased in the epidermis of spot sites as compared with at non-spot sites. Thus, it was surmised that the formation of spots could be predicted by using this gene as indicator. In particular, increases in the expression of MCP-2 gene were observed in the spot model mice not only during UV radiation, but were also observed during the discoloration period when the brown color of the epidermis began to discolor due to UV radiation following completion of UV radiation, and during the pigment spot formation period when spots subsequently began to appear and be formed. Since the expression of this gene in the epidermis of the spot model mice also increases during the discoloration period, the future formation of spots could be predicted during the discoloration period before spots have formed.

### Spot Model Mice

Spot model mice can be produced as described in the previously listed publication by M. Naganuma et al, op. cit. In short, about 7 week old mice are irradiated with ultraviolet rays about 3 times a week for about 8 weeks at an intensity of about 99 mJ/cm² under an ultraviolet light source (Toshiba FL-SE: UVB). Uniform pigmentation of the skin (browning of the skin) is observed during this UV radiation period. This pigmentation nearly completely disappears about 2 weeks after UV radiation has been discontinued (discoloring period). Subsequently, small, light brown pigment spots having a diameter of about 2mm or less (so-called age spot-like spots) began to appear (pigment spot appearance and formation period).

### Skin Test Method for Predicting Spot Formation

The present invention provides a skin test method for predicting the formation of spots in skin, and preferably human skin. This method judges skin to be susceptible to spot formation in the case expression in the epidermis of MCP-2 gene is increased as compared with normal expression in the epidermis. The evaluation criterion may be, for example, judging skin to be susceptible to spot formation if the expression in the epidermis of MCP-2 gene is increased by at least 10%, at least 20%, at least 30%, at least 50%, at least 70% or at least 100% in comparison with the expression of those genes in a control epidermis. The skin to be tested may be, for example, skin of the face, neck, limbs or any other portion of the skin that is susceptible to the formation of spots or for which there is concern over the formation of spots. The normal epidermis that is free of spot formation, namely the control epidermis, may be epidermis from the same individual that is, for example, not likely to be exposed to ultraviolet rays or from a site that is relatively resistant to the formation of skin spots such as the abdomen or thigh.

Increases in gene expression in the epidermis are determined by, for example, measuring the amount of protein encoded by the genes in the epidermis. For example, an increase in the expression of MCP-2 in the epidermis is determined by measuring the amount of MCP-2 in the epidermis. Preferably, this measurement uses a specific antibody to the aforementioned protein, and can be carried out by various known methods in the industry, such as immunostaining methods using fluorescent substances, pigments or enzymes, Western blotting or immunoassay methods such as ELISA and RIA. In addition, increases in expression can also be determined by extracting RNA from the epidermis and measuring the amount of mRNA that encodes the gene. Extraction of mRNA and measurement of the amount thereof are carried out by known methods in the industry, and for example, quantification of RNA is carried out by the quantitative polymerase chain reaction (PCR) method.

Expression in the epidermis of a polynucleotide capable of hybridizing under highly stringent conditions to the aforementioned genes can be determined by extracting RNA from the epidermis and measuring the amount of mRNA corresponding to the polynucleotide. Extraction of mRNA and measurement of an amount thereof are known in the industry, and for example, quantification of RNA is carried out by a quantitative polymerase chain reaction (PCR) method.

As has been previously described, the present invention is based on the finding that, as a result of conducting a microarray analysis of respective epidermal RNA from spot sites and non-spot sites in spot model mice irradiated with ultraviolet rays, the expression of MCP-2 gene is specifically increased in the epidermis of spot sites as compared with non-spot sites. Thus, it is surmised that a medicament could be developed that inhibits the formation of spots and/or removes formed spots by using as an indicator inhibition of the expression of the above gene in the epidermis and/or the activity of gene products thereof in the form of the aforementioned protein, for example, inhibition of the expression of MCP-2 gene in the epidermis and/or the activity of MCP-2.

Thus, the present disclosure provides a pharmaceutical or skin external composition comprising an inhibitor for inhibiting expression of the aforementioned gene as a spot formation inhibitory factor and/or spot removal factor. The composition is able to prevent the formation or remove spots in the skin.

Examples of inhibitors for inhibiting the activity of MCP-2 include amino terminal-cleaved MCP-2 having chemokine antagonist activity which lacks the NH₂-terminal amino acid sequence equivalent to amino acid No. 1, Nos. 1 to 2, Nos. 1 to 3, Nos. 1 to 4 or Nos. 1 to 5 of the naturally-occurring MCP-2 described in Japanese Unexamined Patent Publication (Kohyo) No. 2001-518296.

In addition, MCP-2 inhibitors include CCR-1, -3 or - 5 receptor antagonists known to be bonded by MCP-2. CCR-3 receptor antagonists are described in, for example, Japanese Unexamined Patent Publication (Kokai) No. H11-14782, Japanese Unexamined Patent Publication (Kohyo) No. 2002-512957, Japanese Unexamined Patent Publication (Kokyo) No. 2002-512960, Japanese Unexamined Patent Publication (Kohyo) No. 2002-530374, Japanese Unexamined Patent Publication (Kohyo) No. 2002-512957 and Japanese Unexamined Patent Publication (Kohyo) No. 2003-510248.

Specific examples of these CCR-3 receptor antagonists include:
N-{1-(S)-[4-(3,4-dichlorobenzyl)piperazin-1-ylmethyl]-2-methylpropyl}-4-methylbenzamide dihydrochloride;
N-{1-(S)-[4-(3,4-dichlorobenzyl)piperazin-1-ylmethyl]-2,2-dimethylpropyl}-4-methylbenzamide dihydrochloride;
N-{1-(S)-[4-(3,4-dichlorobenzyl)piperizin-1-ylmethyl]-2-methylpropyl}-4-methylbenzamide dihydrochloride;
N-{1-(R)-[4-(3,4-dichlorobenzyl)piperizin-1-ylmethyl]-2-methylpropyl}-4-(2-aminoethyl)benzamide dihydrochloride;
N-{1-(R)-[4-(3,4-dichlorobenzyl)piperizin-1-ylmethyl]-2-methylpropyl}-5-methylthiophene-2-carboxamide hydrochloride;
1-{1-(R)-[4-(3,4-dichlorobenzyl)piperazin-1-ylmethyl]-2-methylpropyl}-3-(3-methoxyphenyl) urea;
1-{1-(R)-[4-(3,4-dichlorobenzyl)piperizin-1-ylmethyl]-2-methylpropyl}-3-(3-methoxyphenyl) urea;
(S)-ethyl-2-(4-methylbenzenesulfonylamino)-3-(4-hydroxyphenyl)propionate;
(S)-ethyl-2-(5-dimethylaminonapthalene-1-sulfonylamino)-3-(4-hydroxyphenyl)propionate;
(S)-ethyl-2-(naphthalene-2-sulfonylamino)-3-(4-hydroxyphenyl)propionate;
(S)-ethyl-2-(thiophene-2-sulfonylamino)-3-(4-hydroxyphenyl)propionate;
(S)-ethyl-2-(quinoline-8-sulfonylamino)-3-(4-hydroxyphenyl)propionate;
(S)-ethyl-2-(2,4,6-trimethylbenzenesulfonylamino)-3-(4-hydroxyphenyl)propionate;
(S)-ethyl-2-(4-bromobenzenesulfonylamino)-3-(4-hydroxyphenyl)propionate;
(S)-ethyl-2-(4-chlorobenzenesulfonylamino)-3-(4-hydroxyphenyl)propionate;
(S)-ethyl-2-(4-methoxybenzenesulfonylamino)-3-(4-hydroxyphenyl)propionate;
(S)-ethyl-2-methanesulfonylamino)-3-(4-hydroxyphenyl) propionate;
(S)-ethyl-2-[2-(E)-styrylsulfonylamino]-3-(4-hydroxyphenyl)propionate;
(S)-ethyl-2-(3-trifluoromethylbenzenesulfonylamino)-3-(4-hydroxyphenyl)propionate;
(S)-ethyl-2-(2,5-dichlorothiophene-3-sulfonylamino)-3-(4-hydroxyphenyl)propionate;
(S)-ethyl-2-(2-bromobenzenesulfonylamino)-3-(4-hydroxyphenyl)propionate;
(S)-ethyl-2-[5-(2-pyridyl)thiophene-2-sulfonylamino]-3-(4-hydroxyphenyl)propionate;
(S)-ethyl-2-(1,3-dimethyl-5-chloro-2-pyrazoline-4-sulfonylamino)-3-(4-hydroxyphenyl)propionate;
(S)-ethyl-2-(4-biphenylsulfonylamino)-3-(4-hydroxyphenyl)propionate;
(S)-ethyl-2-(2-nitro-4-methoxybenzenesulfonylamino)-3-(4-hydroxyphenyl)propionate;
(S)-ethyl-2-(2,5-dichlorobenzenesulfonylamino)-3-[4-(2,5-dichlorobenzenesulfonyloxy)phenyl]propionate;
(S)-ethyl-2-(2,4-difluorobenzenesulfonylamino)-3-[4-(2,4-difluorobenzenesulfonyloxyphenyl)propionate;
(S)-ethyl-2-(5-dimethylaminonaphthalene-1-sulfonylamino)-3-(4-hydroxyphenyl)propionate;
(S)-ethyl-2-(thiophene-2-sulfonylamino)-3-(4-hydroxyphenyl)propionate:
(S)-ethyl-2-(2,4,6-trimethylbenzenesulfonylamino)-3-(4-hydroxyphenyl)propionate;
(S)-ethyl-2-(4-bromobenzenesulfonylamino)-3-(4-hydroxyphenyl)propionate;
(S)-ethyl-2-(4-chlorobenzenesulfonylamino)-3-(4-hydroxyphenyl)propionate;
(S)-ethyl-2-(4-methoxybenzenesulfonylamino)-3-(4-hydroxyphenyl)propionate;
(S)-ethyl-2-[2-(E)-styrylsulfonylamino]-3-(4-hydroxyphenyl)propionate;
(S)-ethyl-2-(3-trifluoromethylbenzenesulfonylamino)-3-(4-hydroxyphenyl)propionate;
(S)-ethyl-2-(2,5-dichlorothiophene-3-sulfonylamino)-3-(4-hydroxyphenyl)propionate;
(S)-ethyl-2-(2-bromobenzenesulfonylamino)-3-(4-hydroxyphenyl)propionate;
(S)-ethyl-2-[5-(2-pyridyl)thiophene-2-sulfonylamino]-3-(4-hydroxyphenyl)propionate;
(S)-ethyl-2-(2-nitro-4-methoxybenzenesulfonylamino)-3-(4-hydroxyphenyl)propionate;
(S)-ethyl-2-(2,5-dichlorobenzenesulfonylamino)-3-[4-(2,5-dichlorobenzenesulfonyloxy)phenyl]propionate;
(S)-ethyl-2-(2,5-dichlorothiophene-3-sulfonylamino)-3-(4-hydroxyphenyl)propionate;
(S)-ethyl-2-(2-bromobenzenesulfonylamino)-3-(4-hydroxyphenyl)propionate;
(S)-ethyl-2-(2,5-dichlorobenzenesulfonylamino)-3-[4-(2,5-dichlorobenzenesulfonyloxy)phenyl]propionate;
(S)-ethyl-2-(1-naphthoylamino)-3-(4-nitrophenyl) propionate;
(S)-isopropyl-2-(1-naphthoylamino)-3-(4-nitrophenyl) propionate;
(S)-methyl-2-(1-naphthoylamino)-3-(4-nitrophenyl) propionate;
(S)-benzyl-2-(1-naphthoylamino)-3-(4-nitrophenyl) propionate;
(S)-ethyl-2-(1-naphthoylamino)-3-(4-chlorophenyl) propionate;
(S)-ethyl-2-benzoylamino-3-(4-hydroxyphenyl) propionate;
(S,S)-ethyl-2-(2-benzylxycarbonylamino-3-phenylpropionylamino)-3-(4-hydroxyphenyl)propionate;
(S,S)-ethyl-2-(N-acetylpyrrolidine-2-benzoylamino)-3-(4-hydroxyphenyl)propionate;
(S)-ethyl-2-cyclohexanylamino-3-(4-hydroxyphenyl) propionate;
(S)-ethyl-2-(3,3-diphenylpropionylamino)-3-(4-hydroxyphenyl)propionate;
(S)-ethyl-2-(3-phenylpropionylamino)-3-(4-hydroxyphenyl)propionate;
(S)-ethyl-2-[2-(2-napthyl)acetylamino]-3-(4-hydroxyphenyl)propionate;
(S)-ethyl-2-(4-phenylbutyrylamino)-3-(4-hydroxyphenyl)propionate;
(S)-ethyl-2-pentanylamino-3-(4-hydroxyphenyl) propionate;
(S)-ethyl-2-pentanylamino-3-(4-hydroxyphenyl) propionate;
(S)-ethyl-2-(4-benzoylbenzoylamino)-3-(4-hydroxyphenyl)propionate;
(S)-ethyl-2-(2-furanyl)amino-3-(4-hydroxyphenyl) propionate;
(S)-ethyl-2-(1-naphthoylamino)-3-(4-hydroxyphenyl) propionate;
(S)-ethyl-2-(5-hydroxyindonyl)amino-3-(4-hydroxyphenyl)propionate;
(S)-ethyl-2-piperonylamino-3-(4-hydroxyphenyl) propionate;
(S)-ethyl-2-picolinylamino-3-(4-hydroxyphenyl) propionate;
(S)-ethyl-2-(3-nitro-4-chlorobenzoylamino)-3-(4-hydroxyphenyl)propionate;
(S)-ethyl-2-(3-hydroxy-4-nitrobenzoylamino)-3-(4-hydroxyphenyl)propionate;
(S)-ethyl-2-(8-quinolinylamino)-3-(4-hydroxyphenyl) propionate;
(S)-ethyl-2-benzoylamino-3-phenylpropionate;
(S)-methyl-2-benzoylamino-3-(4-hydroxyphenyl) propionate;
(S)-benzyl-2-benzoylamino-3-(4-hydroxyphenyl) propionate;
(S)-ethyl-2-benzoylamino-3-(4-methoxyphenyl) propionate;
(S)-ethyl-2-tert-butyloxycarbonylamino-3-(4-nitrophenyl)propionate;
(S)-ethyl-2-tert-butyloxycarbonylamino-3-(4-aminophenyl)propionate;
(S)-ethyl-2-benzoylamino-3-(3,5-diiodo-4-hydroxyphenyl)propionate;
(S)-ethyl-2-carboxybenzoylamino-3-(4-hydroxyphenyl) propionate;
(S)-ethyl-2-benzoylamino-3-(1-naphthyl)propionate;
(±)-ethyl-2-benzolylamino-3-[3-(benzoyloxy)phenyl] propionate;
(±)-ethyl-2-benzoylamino-3-(3-hydroxyphenyl)propionate;
(R,S)-ethyl-2-benzoylamino-3-(2-hydroxyphenyl) propionate;
(S)-ethyl-2-benzoylamino-3-(4-aminophenyl)propionate;
(S)-ethyl-2-benzoylamino-3-(4-nitrophenyl)propionate;
(S)-ethyl-2-(2-phenylacetylamino)-3-(4-hydroxyphenyl) propionate;
(S)-ethyl-2-benzoylamino-3-(3-indoyl)propionate;
(±)-ethyl-2-(benzolylamino)-2-phenylacetate;
(±)-ethyl-2-(benzoylamino)-4-phenylbutyrate;
(S)-ethyl-2-(1-naphthoylamino)-3-(4-hydroxyphenyl) propionate;
(S)-ethyl-2-benzoylamino-3-(4-hydroxyphenyl)propionate;
(S)-ethyl-2-cyclohexanylamino-3-(4-hydroxyphenyl) propionate;
(S)-ethyl-2-(1-naphthoylamino)-3-(4-hydroxyphenyl) propionate;
(S)-benzyl-2-benzoylamino-3-(4-hydroxyphenyl) propionate;
(S)-ethyl-2-benzoylamino-3-(4-methoxyphenyl)propionate;
(S)-ethyl-2-benzoylamino-3-(1-napthyl)propionate;
(±)-ethyl-2-benzolylamino-3-[3-(benzoyloxy)phenyl] propionate;
(±)-ethyl-2-benzoylamino-3-(3-hydroxyphenyl)propionate;
(R,S)-ethyl-2-benzoylamino-3-(2-hydroxyphenyl) propionate;
(S)-ethyl-2-benzoylamino-3-(4-nitrophenyl)propionate;
(±)-ethyl-2-(benzoylamino)-2-phenylacetate;
(±)-ethyl-2-(benzoylamino)-4-phenylacetate;
(S)-ethyl-2-(1-naphthoylamino)-3-(4-nitrophenyl) propionate;
(S)-ethyl-2-(1-naphthoylamino)-3-(4-hydroxyphenyl) propionate; and,
(S)-ethyl-2-benzoylamino-3-(4-nitrophenyl)propionate.

Examples of CCR-1 receptor antagonists include the compounds described in International Publication WO 97/24325; WO 98/38167 by Pfizer, Inc., WO 97/44329 by Teijin Co., Ltd.; WO 98/04554 by Banyu Pharmaceutical Co., Ltd.; WO 98/27815, WO 98/25604, WO 98/25605, WO 98/25617 and WO 98/31364 by Merck & Co., Inc.; WO 98/02151 and WO 99/37617 by LeukoSite, Inc.; WO 99/37651 and WO 99/37619 by LeukoSite, Inc.; US Provisional Patent Application No. 60/021,716 (filed on July 12, 1996); US Patent Application No. 09/146,827 and 09/148,236 (filed on September 4, 1998); Hesselgesser et al., J. Biol. Chem. 273(25): 15687-15692 (1998); and Howard et al., J. Medicinal Chem. 41(13): 2184-2193 (1998).

In addition, an example of a CCR-5 receptor antagonist is described in Japanese Unexamined Patent Publication (Kohyo) No. 2002-543186.

A pharmaceutical or skin external composition is applied in the form of, for example, an aqueous solution, oily liquid, other type of solution, milky liquid, cream, gel, suspension, microcapsules, powder, granules, capsules or solid preparation. After preparing in these forms using conventionally known methods, they can be applied, attached, sprayed, injection, consumed or inserted into the body in the form of a lotion, milky lotion, cream, ointment, salve, poultice, aerosol, water-oil two-layer system, water-oil-powder three-layer system, injection, oral preparation (e.g., tablets, powders, granules, pills, syrup, lozenges) or suppositories. The aforementioned inhibitor can be contained in this composition at, for example, 0.001 mM to 1 M, preferably 0.01 to 100 mM and more preferably 0.1 to 10 mM, based on the total amount of the composition with any particular limitations as a spot formation inhibitory factor and/or spot removal factor.

Among these drug forms, lotions, milky lotions, creams, ointments, salves, poultices, aerosols and other skin external preparations are suitable. Furthermore, the skin external preparations listed here include prescription pharmaceuticals, over-the-counter pharmaceuticals (such as ointments) and cosmetics (such as facial washes, milky liquids, creams, gels, essences (beauty washes), facial packs, facial masks and other basic cosmetics, foundations, lipstick and other makeup cosmetics, as well as oral cavity cosmetics, fragrant cosmetics, hair cosmetics and body cosmetics). A pharmaceutical or skin external preparation is particularly suitably applied as a spot preventive cosmetic.

Conventionally known vehicles and fragrances as well as oils, surfactants, antiseptics, metal ion chelating agents, water-soluble polymers, thickeners, pigments and other powdered components, ultraviolet protectors, moisturizers, antioxidants, pH regulators, cleansers, desiccants or emulsifiers and so forth are suitably incorporated in a pharmaceutical or skin external preparation corresponding to the desired drug form. Moreover, other pharmacologically active components can be incorporated into a pharmaceutical or skin external preparation within a range that does not impair the expected effects as a result of incorporation.

### Spot Formation Inhibitory Factor and/or Spot Removal Factor Screening Method

The present invention additionally provides a method for screening for a spot formation inhibitory factor and/or spot removal factor. This method is characterized by evaluating a candidate compound for the ability to inhibit the expression and/or activity in the epidermis of the MCP2 gene, and select an inhibitor having this inhibitory ability as a spot formation inhibitory factor and/or spot removal factor.

In a preferable aspect thereof, the aforementioned screening method comprises applying an inhibitor having the inhibitory ability to a non-human spot model animal, and selecting an inhibitor having a spot formation inhibitory effect and/or spot removal effect.

A process for confirming the spot formation inhibitory effect and/or spot removal effect of the inhibitor can be carried out by using a non-human model animal such as a non-human spot model animal. Examples of animals that can be used as models other than mice include rats, rabbits and various other non-human animals. In a preferable aspect thereof, a solution such as an aqueous solution of the inhibitor is prepared, and then repeatedly applied to the skin of the non-human spot model animal followed by evaluation of spot formation to judge the presence or absence of the above effects.

The following provides a more detailed explanation of the present invention using a specific example. It is to be noted that disclosures relating to genes other than MCP2 are not part of the present invention. Furthermore, the present invention is not limited thereto.

### Production of Spot Model Mice

Spot model mice were produced as described in M. Naganuma et al, op cit. In short, 7 week old mice were irradiated with ultraviolet rays 3 times a week for 8 weeks (UV radiation period) at an intensity of 99 mJ/cm² under an ultraviolet light source (Toshiba FL-SE: UVB). The animals underwent a discoloring period from age 15 to 23 weeks (until about 8 weeks following completion of UV radiation period), a pigment spot appearance period from age 23 to 35 weeks (about 18 to 30 weeks after completion of UV radiation period), and a pigment spot formation period from age 35 to 52 weeks (about 30 to 47 weeks after completion of UV radiation period).

### Sampling of RNA from Skin

The entire layer of skin was sampled from the back of each mouse followed by removal of the fatty layer and separation of the epidermis by heating. RNA was extracted from the epidermis using ISOGEN (Nippon Gene, manufacturer's recommended protocol) and purified using RNeasy (Qiagen). The dermis was cut into 1 centimeter squares, frozen in liquid nitrogen and crushed followed by extraction of RNA using ISOGEN (Nippon Gene, manufacturer's recommended protocol) and purification using RNeasy (Qiagen). The resulting RNA was phoresed in TBE agarose gel and stained with SYBR Green (Molecular Probes) to confirm quality and the degree of purification.

Samples during the UV radiation period were collected from about 10 week old mice, and controls for these samples were collected from about 14 week old mice. Samples during the discoloring period were collected from about 20 week old mice, and controls for these samples were collected from about 20 week old mice. Samples during the pigment spot formation period were collected from about 42 week old mice, and controls for these samples were collected from about 40 week old mice.

### Reaction of Microarray Samples

Gene chips manufactured by Affymetrix Japan were used for microarray analyses.

### Affymetrix Sample Preparation:

The reaction was carried out according to the Affymetrix recommended protocol. 10 µg of RNA were reacted with 100 pmol of oligo-dT primer (24 mer) (Sigma) for 10 minutes at 70°C. Subsequently, 4 µl of 5x first strand reaction buffer, 2 µl of 0.1 M DTT, 1 µl of 10 mM dNTP and 2 µl of Superscript II (all Invitrogen products) were added and reacted for 1 hour at 42°C. 91 µl of RNase-free water, 30 µl of 5x second strand reaction buffer, 3 µl of 10 mM dNTP mix, 1 µl of 10 U/µl E. coli DNA ligase, 14 µl of 10 U/µl E. coli DNA polymerase and 1 µl of 2 U/µl RNaseH (all Invitrogen products) were then added and reacted for 2 hours at 16°C. Moreover, 2 µl of T4 DNA polymerase (10 U/µl) (Invitrogen) were added and after reacting for 5 minutes at 16°C, 10 µl of 0.5 M EDTA were added to stop the reaction. Cleanup was carried out using Phase Lock Gels (Qiagen) and eluted into 12 µl of RNase-free water. Biotin-added cRNA was produced by an in vitro transcription reaction from the resulting cDNA (Enzo, Farmingdale), and then purified using RNeasy (Qiagen). After fragmenting the cRNA for 35 minutes at 94°C in a fragmentation buffer, the resulting fragmented cRNA was used for gene chip hybridization. After washing the chip, the data was read using a scanner manufactured by Affymetrix.

### Analysis Results

As a result of comprehensively analyzing about 9,000 types of genes, the expression of AK12157 gene, MCP-2 gene and Gene Groups (1) to (3) were found to be remarkably increased in epidermis of the spot model mice as compared with the epidermis of control mice. Particularly interestingly, increased expression of AK012157 gene and MCP-2 gene was observed not only during the UV radiation period, but was also continuously observed during the discoloration period, when the brown color of the epidermis began to discolor due to the UV radiation following completion of UV radiation, and during the subsequent pigment spot formation period when spots began to appear and be formed. Since the expression of these genes was also increased during the discoloration period prior to the formation of spots in the epidermis of the model mice, the use of these genes as indicators would make it possible to predict the future formation of spots even during the discoloration period prior to spot formation. Those results are shown in Tables 1 and 2 below.

**Table 1 Fluctuation Rates of Each Gene Based on Control**

| | UV radiation period | Discoloration period | Pigment spot formation period |
|---|---|---|---|
| MCP-2 | 4.7 | 13.8 | 3.2 |
| AK012157 | 5.3 | 5.5 | 6.4 |

(Expression ratio based on a value of 1 for a normal site or non-UV irradiated site. Average values for n = 2 for the UV radiation and discoloration periods, and n = 3 for the pigment spot formation period.)

**Table 2**

| Gene Name | Spot sites/non-spot sites (n=4) |
|---|---|
| Mcp9 | 3.9 |
| Mcp10 | 3.6 |
| Isg15 | 4.1 |
| Usp18 | 2.2 |
| Oas12 | 4.1 |
| Gbp2 | 3.3 |
| Gtpi | 3.2 |
| Ifi47 | 2.8 |
| Igtp | 2.7 |
| Tgtp | 2.6 |
| Sprr2A | 23.3 |
| Krt2-6b | 3.4 |
| Cdk5rap2 | 12.4 |
| Mef2C | 4.8 |
| Gsta4 | 3.0 |
| Osf2 | 2.9 |
| Tnc | 2.9 |
| Igfbp6 | 2.9 |
| Ppicap | 2.6 |
| Mcp-6 | 1.8 |
| Mm. 74656 | 2.9 |

### RT-PCR

1 µg each of the RNA collected from epidermis (epidermis and dermis of a spot site and non-spot site) was reacted for 10 minutes at 70°C with 100 pmol of oligo-dT primer (24 mer) (Sigma) (total volume: 20 µl). Subsequently, 4 µl of 5x first strand reaction buffer, 2 µl of 0.1 M DTT, 4 µl of 2.5 mM dNTP mix, and 1 µl of Superscript II (all Invitrogen products) were added and reacted for 1 hour at 42°C. Finally, an elongation reaction was carried out for 10 minutes at 70°C to prepare cDNA templates. 5 µl of rTaq 10x buffer, 3 µl of 25 mM MgCl₂, 5 µl of 2.0 mM dNTP mix, 0.5 µl of rTaq (all Toyobo), 33.5 µl of ddH₂O, 1 µl of each cDNA template, and 1 µl of each sense and antisense primer (refer to the sequences shown below) at 20 mM each were all added (total volume: 50 µl) followed by carrying out the PCR reaction (30 cycles consisting of 94°C for 2 minutes (94°C for 30 seconds, 55°C for 30 seconds and 72°C for 1 minute) and finally 72°C for 10 minutes).

| MCP-2 Primer Sequences: | |
|---|---|
| Sense | TTCTTTGCCTGCTGCTCATA (SEQ ID NO. 4) |
| Antisense | GACAAGGATGAGAAAACACG (SEQ. ID NO. 5) |

| AK012157 Primer Sequences: | |
|---|---|
| Sense | ACTCCGGCTCCTTCACTATG (SEQ. ID NO. 6) |
| Antisense | CTTTGGAATGAGGACTTGGA (SEQ. ID NO. 7) |

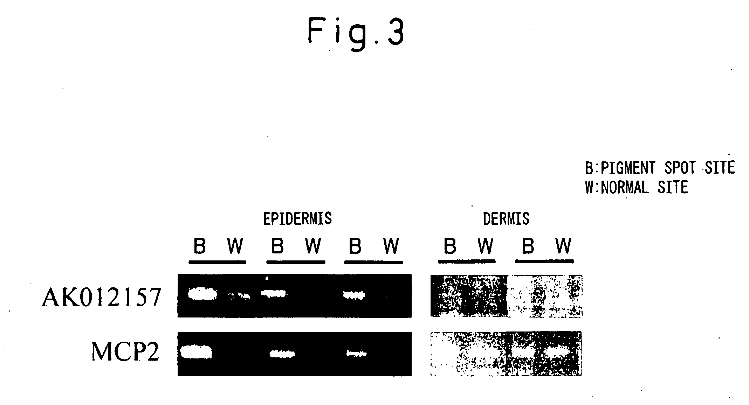

Finally, the PCR products were electrophoresed with agarose gel containing 1.5% ethidium bromide to obtain a band of about 300 bp. This result is shown in Fig. 3. As is clear from Fig. 3, AK012157 gene and MCP-2 gene were significantly expressed at a spot site of the epidermis, but were observed to be hardly expressed at all at a non-spot site of the epidermis. In addition, there were no differences in expression for either gene in the dermis.

### In Situ Hybridization (ISH)

After fixing mouse epidermal tissue with 10% neutral formalin and embedding in paraffin, tissue section slides were produced according to ordinary methods. The slides were then subjected to an in situ hybridization reaction using the HX System Automated Slide Processor manufactured by Ventana Japan (Protocol: Japan Open Blue 8.0). The tissue sections were subjected to pretreatment consisting of blocking and protease treatment, followed by hybridizing for 6 hours at 68°C with 500 ng of a Dig-labeled riboprobe produced from the AK012157 sequence using T7 polymerase. The hybridization product was then washed, and after reacting with Anti-Dig-alkaline phosphatase and washing, the product was stained using NBT/BCIP substrate followed by sealing and observing with a light microscope.

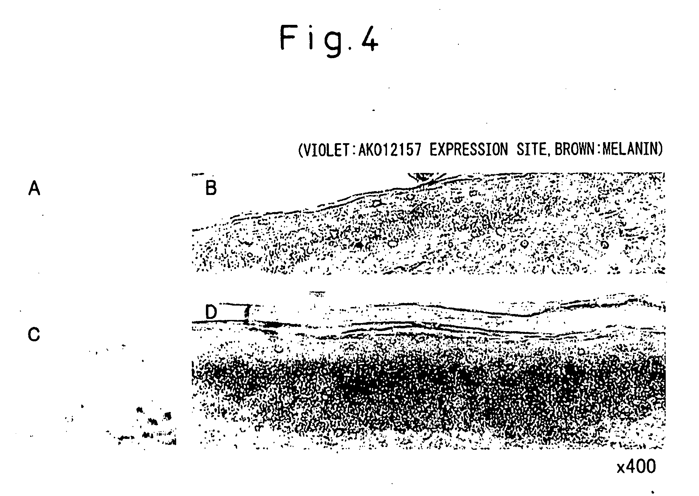

Those results are shown in Fig. 4. As is clear from Fig. 4, AK012157 gene was only expressed significantly in a spot site of the epidermis, and was observed to be hardly expressed at all in a non-spot site of the epidermis.

### Immunohistochemical Staining (IHC)

After fixing mouse epidermal tissue with neutral formalin and embedding in paraffin, tissue sections were produced in accordance with ordinary methods. After then treating for 15 minutes with 1% H₂O₂, the sections were blocked for 30 minutes and reacted overnight with 1/50-diluted anti-mouse MCP2 antibody (R&D Systems). This was detected by tyramide sensitization (TSA System, Perkin-Elmer). After washing, the sections were reacted with secondary antibody with HRP and then washed, followed by reacting with FITC-labeled tyramide, washing, embedding and observed with a fluorescent microscope.

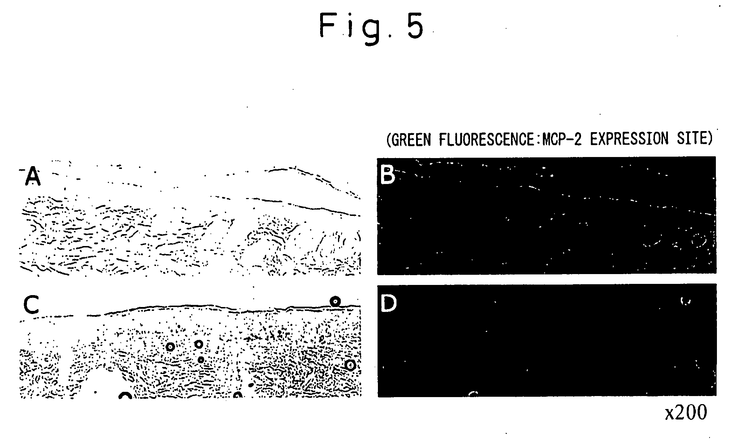

Those results are shown in Fig. 5. As is clear from Fig. 5, MCP-2 gene was observed to be significantly expressed at a spot site in the epidermis, and was observed to hardly be expressed at all at a non-spot site in the epidermis.

### Comparison of Expression of Major Signal Transfer Protein Groups

When epidermal proteins were detected at spot sites and normal sites in spot model mice, and the expressed amounts of the major signal protein groups shown in Fig. 6 were investigated by Western blotting, phosphorylated STAT1 exhibited a particularly large difference in expression between spot sites and normal sites. This result coincide with the finding that an interferon-induced gene group (Gene Group (1)) demonstrates increased expression at spot sites.

### INDUSTRIAL APPLICABILITY

The present invention is able to provide a skin test method for predicting the formation of spots in the skin.

## Claims

1. A skin test method for predicting the formation of melanin-pigmented skin spots, comprising determining expression of Monocyte Chemoattracting Protein 2 (MCP2) in epidermis, and judging skin to be susceptible to the formation of melanin-pigmented skin spots in the case expression of MCP2 in epidermis is increased as compared with normal expression in the epidermis.

2. A method according to claim 1, wherein the formation of melanin-pigmented skin spots is caused by WB radiation.

3. A method according to claim 1 or 2, wherein the increase in the expression of MCP2 in epidermis is determined by measuring the amount of MCP2 in the epidermis.

4. A method according to claim 3, wherein the measurement is carried out by ELISA or RIA using antibody specific to MCP2.

5. A method according to any of claims 1 to 4, wherein the increase in the expression of MCP2 in the epidermis is determined by measuring the amount of mRNA encoding MCP2 extracted from the epidermis.

6. A method according to claim 5, wherein the measurement of the mRNA is carried out by a polymerase chain reaction method.

7. A method for screening for a melanin-pigmented skin spot formation inhibitory factor and/or melanin-pigmented skin spot removal factor, comprising the steps of evaluating a candidate compound for the ability to inhibit the expression and/or activity in the epidermis of MCP2, and selecting an MCP2 inhibitor having this inhibitory ability as a melanin-pigmented skin spot formation inhibitory factor and/or melanin-pigmented skin spot removal factor.

8. A method according to claim 7, wherein the method further comprises application of the MCP2 inhibitor having inhibitory ability to a melanin-pigmented skin spot formation non-human model animal, and selecting an inhibitor that has a melanin-pigmented skin spot formation inhibitory effect and/or melanin-pigmented skin spot removal effect.

## Patentansprüche

1. Hauttestverfahren zur Vorhersage der Bildung von melaninpigmentierten Hautstellen, das das Bestimmen der Expression von Monocyte Chemoattracting Protein 2 (MCP2) in der Epidermis und die Beurteilung, dass Haut im Falle einer im Vergleich zur normalen Expression von MCP2 in der Epidermis erhöhten Expression von MCP2 in der Epidermis für die Bildung von melaninpigmentierten Hautstellen empfänglich ist, umfasst.

2. Verfahren nach Anspruch 1, wobei die Bildung von melaninpigmentierten Hautstellen durch UVB-Strahlung verursacht wird.

3. Verfahren nach Anspruch 1 oder 2, wobei die Zunahme der Expression von MCP2 in der Epidermis durch Messen der Menge von MCP2 in der Epidermis bestimmt wird.

4. Verfahren nach Anspruch 3, wobei die Messung durch ELISA oder RIA unter Verwendung eines für MCP2 spezifischen Antikörpers durchgeführt wird.

5. Verfahren nach einem der Ansprüche 1 bis 4, wobei die Zunahme der Expression von MCP2 in der Epidermis durch Messen der Menge von mRNA mit Codierung für MCP2, die aus der Epidermis extrahiert wurde, bestimmt wird.

6. Verfahren nach Anspruch 5, wobei die Messung der mRNA durch ein Polymerasekettenreaktionsverfahren durchgeführt wird.

7. Verfahren zum Screening auf einen Hemmfaktor der Bildung von melaninpigmentierten Hautstellen und/oder einen Entfernungsfaktor für melaninpigmentierte Hautstellen, wobei das Verfahren die Stufen der Beurteilung einer Kandidatenverbindung in Bezug auf die Fähigkeit zur Hemmung der Expression und/oder Aktivität von MCP2 in der Epidermis und der Auswahl eines MCP2-Inhibitors mit dieser Hemmfähigkeit als Hemmfaktor der Bildung von melaninpigmentierten Hautstellen und/oder Entfernungsfaktor für melaninpigmentierte Hautstellen umfasst.

8. Verfahren nach Anspruch 7, wobei das Verfahren ferner die Applikation des MCP2-Inhibitors mit Hemmfähigkeit an ein nicht-humanes Modelltier mit Bildung von melaninpigmentierten Hautstellen und die Auswahl eines Inhibitors, der eine Hemmwirkung der Bildung von melaninpigmentierten Hautstellen und/oder Entfernungswirkung für melaninpigmentierte Hautstellen aufweist, umfasst.

## Revendications

1. Méthode de test cutané pour prévoir la formation de taches cutanées pigmentées par la mélanine, comprenant la détermination de l'expression de la protéine chimio-attractive des monocytes 2 (MCP2) dans l'épiderme, et l'estimation que la peau est sensible à la formation de taches cutanées pigmentées par la mélanine dans le cas où l'expression de MCP2 dans l'épiderme est augmentée comparativement à l'expression normale dans l'épiderme.

2. Méthode suivant la revendication 1, dans laquelle la formation de taches cutanées pigmentées par la mélanine est provoquée par irradiation avec des rayons UVB.

3. Méthode suivant la revendication 1 ou 2, dans laquelle l'augmentation de l'expression de MCP2 dans l'épiderme est déterminée en mesurant la quantité de MCP2 dans l'épiderme.

4. Méthode suivant la revendication 3, dans laquelle la mesure est effectuée par analyse ELISA ou RIA en utilisant un anticorps spécifique de MCP2.

5. Méthode suivant l'une quelconque des revendications 1 à 4, dans laquelle l'augmentation de l'expression de MCP2 dans l'épiderme est déterminée en mesurant la quantité d'ARNm codant pour MCP2 extraite de l'épiderme.

6. Méthode suivant la revendication 5, dans laquelle la mesure de l'ARNm est effectuée par une méthode de réaction en chaîne avec une polymérase.

7. Méthode pour sélectionner un facteur inhibiteur de la formation de taches cutanées pigmentées par la mélanine et/ou un facteur d'élimination des taches cutanées pigmentées par la mélanine, comprenant les étapes consistant à évaluer un composé candidat en ce qui concerne l'aptitude à inhiber l'expression et/ou l'activité de MCP2 dans l'épiderme, et à sélectionner un inhibiteur de MCP2 présentant cette capacité inhibitrice comme facteur inhibiteur de la formation de taches cutanées pigmentées par la mélanine et/ou facteur d'élimination des taches cutanées pigmentées par la mélanine.

8. Méthode suivant la revendication 7, qui comprend en outre l'application de l'inhibiteur de MCP2 ayant une capacité inhibitrice à un animal non humain servant de modèle de formation de taches cutanées pigmentées par la mélanine, et la sélection d'un inhibiteur qui présente un effet inhibiteur de la formation de taches cutanées pigmentées par la mélanine et/ou un effet d'élimination des taches cutanées pigmentées par la mélanine.
